(19) Europäisches Patentamt
European Patent Office
Office européen des brevets

(11) Veröffentlichungsnummer: **0 066 211**
**B1**

(12) # EUROPÄISCHE PATENTSCHRIFT

(45) Veröffentlichungstag der Patentschrift: **12.09.84**

(51) Int. Cl.³: **C 07 C 85/24,** C 07 C 87/40

(21) Anmeldenummer: **82104442.7**

(22) Anmeldetag: **21.05.82**

(54) **Katalytische Hydrierung von Di-(4-aminophenyl)methan.**

(30) Priorität: **01.06.81 US 268979**

(43) Veröffentlichungstag der Anmeldung: **08.12.82 Patentblatt 82/49**

(45) Bekanntmachung des Hinweises auf die Patenterteilung: **12.09.84 Patentblatt 84/37**

(84) Benannte Vertragsstaaten: **BE DE FR GB IT NL**

(56) Entgegenhaltungen: **US - A - 3 347 917**
**US - A - 3 742 049**

(73) Patentinhaber: **Mobay Chemical Corporation, Penn Lincoln Parkway West, Pittsburgh, Pennsylvania 15205 (US)**

(72) Erfinder: **Allen, Gary F., 811 Clearview Terrace, New Martinsville WV 26155 (US)**

(74) Vertreter: **Drope, Rüdiger, Dr. et al, c/o Bayer AG Zentralbereich Patente Marken und Lizenzen, D-5090 Leverkusen 1, Bayerwerk (DE)**

EP 0 066 211 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Bei der Herstellung von Di-(4-aminocyclohexyl)methan durch katalytische Hydrierung von Di-(4-aminophenyl)methan werden im wesentlichen drei Stereoisomere gebildet:

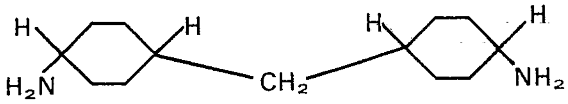

CIS CIS

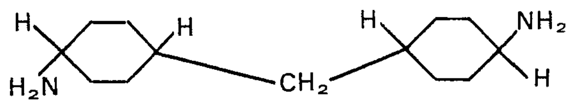

CIS TRANS

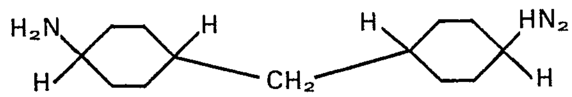

TRANS TRANS

Es ist in der Fachwelt bekannt, dass für die Herstellung eines entsprechenden Isocyanats (mittels des bekannten Verfahrens der Phosgenierung), das bei Raumtemperatur (d.h. von 20°C bis 25°C) flüssig und lagerbeständig ist, die Mischung der für die Phosgenierung eingesetzten Amin-Stereoisomeren das trans-trans-Stereoisomere nur in relativ kleineren Mengen (typischerweise von 15 bis 40 Gew.-%, vorzugsweise von 18,5 bis 23,5 Gew.-%) enthalten darf.

In der Fachwelt sind zahlreiche Verfahrensweisen zur Herstellung solcher Amin-Gemische bekannt, die die geforderte Menge des trans-trans-Isomeren enthalten. Repräsentativ für diese Verfahrensweisen sind diejenigen, die in den US-PSen 3153088, 3155724, 3393236, 3644522, 3711550 und 3766272 beschrieben sind. Diese bekannten Methoden erfordern im allgemeinen die Abtrennung eines die geforderte Menge des trans-trans-Isomeren enthaltenden Amin-Gemisches aus einem Amin-Gemisch, das durch die Hydrierung gebildet wird und etwa 50 Gew.-% des trans-trans-Isomeren enthält. In der Fachwelt sind auch Verfahren zur direkten Herstellung eines die geforderte Menge des trans-trans-Isomeren enthaltenden Amin-Gemisches aus Di-(4-aminophenyl)methan bekannt, die die Zwischenstufe der Abtrennung entbehrlich machen (vgl. die US-PS 2606928). Jedoch sind hierbei die Reaktionsgeschwindigkeiten für Zwecke technischer Anwendungen viel zu niedrig.

Zahlreiche Verfahren zur Herstellung von Di-(4-aminocyclohexyl)methan aus Di-(4-aminophenyl)methan mittels katalytischer Hydrierung unter Verwendung von Ruthenium-Katalysatoren in Form von Trägerkatalysatoren oder trägerfreien Katalysatoren sind in der Fachwelt bekannt. Typische Verfahren dieser Art sind in den US-PSen 2494563, 2606924, 2606928, 2606925, 3347917, 3676495, 3959374, 3743677, 3914307, 3825586, 3636108 und 4161492 beschrieben. Wenngleich einige dieser Verfahren ein Amin-Gemisch liefern, das das trans-trans-Isomer in einer Menge enthält, die die Herstellung flüssiger, lagerbeständiger Isocyanate ermöglicht, sind die Reaktionsgeschwindigkeiten viel zu niedrig für einen technischen Einsatz.

Es wurde auch beschrieben, dass Katalysatoren auf Ruthenium-Basis für die Hydrierung von

(a) polycycloaromatischen Polyaminen, die aus Anilin und Formaldehyd aufgebaut sind (vgl. die US-PS 4226737),
(b) 2,4-Bis(p-aminobenzyl)anilin (vgl. die US-PS 3557180),
(c) 2,4'-Diaminodiphenylmethan (vgl. die US-PS 3590002) und
(d) Tolylendiamin/Formaldehyd-Kondensaten (vgl. die US-PSen 3330850 und 3361814)

geeignet sind. Keines dieser Verfahren betrifft jedoch das hier vorliegende Problem, d.h. die Herstellung von Di-(4-aminocyclohexyl)methan, das das trans-trans-Isomer in der erforderlichen Menge enthält.

Schliesslich wurde noch die Verwendung einer speziellen amorphen Form des Rutheniumoxids für die Herstellung von Di-(4-aminocyclohexal)-methan aus Di-(4-nitrophenyl)methan beschrieben (vgl. die US-PS 3742049).

Die vorliegende Erfindung betrifft ein Verfahren zur Herstellung flüssigen und 15 bis 40 Gew.-%, vorzugsweise 18,5 bis 23,5 Gew.-%, des trans-trans-Isomeren enthaltenden Di-(4-aminocyclohexyl)methans aus Di-(4-aminophenyl)methan unter Verwendung eines trägerfreien Rutheniumdioxid-Katalysators und Einhaltung spezieller Verfahrensbedingungen. Im einzelnen sind während der Hydrierung ein Wasserstoff-Druck von mindestens 172,4 bar (2500 psi) und eine Temperatur im Bereich von 150°C bis 210°C einzuhalten.

Die verwendeten Katalysatoren sind allgemein bekannt und im Handel erhältlich. Die zur Zeit bevorzugten Katalysatoren sind zwei hydratisierte Rutheniumdioxid-Katalysatoren, die von der Firma Engelhard bezogen werden können (wobei der eine 40,9% Ruthenium und der andere 58,3% Ruthenium enthält), und hydratisiertes Rutheniumdioxid von Johnson-Matthey.

Bei der Durchführung des Verfahrens gemäss der vorliegenden Erfindung gelangen die allgemein in der Fachwelt angewandten Verfahrensweisen zum Einsatz, wobei die einzigen besonderen Anforderungen in den im Vorstehenden angegebenen Bedingungen für Druck und Temperatur bestehen. Die Hydrierung wird vorzugsweise in Gegenwart eines inerten Lösungsmittels, das den angestrebten Verlauf der Hydrierung praktisch nicht stört.

Geeignete Lösungsmittel sind Ether wie Isopropylether oder n-Butylether, Cyclohexan oder andere aliphatische Kohlenwasserstoffe, Alkohole wie Butylalkohol, Methanol, Ethanol, Isopropanol und Propanol sowie cyclische Ether wie Tetrahydrofuran und Dioxan. Die Menge des eingesetzten Lösungsmittels kann von 0 bis 95 Gew.-%, bezogen auf das Gesamtgewicht von Aminen und Lösungsmittel, betragen. Vorzugsweise wird die

Menge des verwendeten Lösungsmittels so bemessen, dass die Konzentration des Ausgangs-Diamins in der Reaktionsmischung etwa 5 bis 50 Gew.-% beträgt. Das gegenwärtig bevorzugte Lösungsmittel ist n-Butylether.

Der Katalysator wird in einer Lösung des Ausgangs-Diamins suspendiert, und die entstandene Suspension wird in einem geeigneten Hydriergefäss der Hydrierung unterworfen. Es wird eine solche Katalysator-Menge verwendet, das die Menge in der Reaktionsmischung vorhandenen Rutheniums vorzugsweise mindestens 0,05 Gew.-%, bezogen auf das Gewicht des Ausgangs-Diamins, beträgt. Besonders bevorzugt wird ein Ruthenium-Gehalt im Bereich von etwa 0,1 bis 3 Gew.-%, bezogen auf das in den Reaktionsmischungen vorhandene Ausgangs-Diamin. Wirtschaftliche Überlegungen bestimmen im allgemeinen die einzusetzende Höchstmenge an Katalysator, da dieser im allgemeinen teuer ist. Vorzugsweise liegt die eingesetzte Katalysator-Menge im Bereich von etwa 0,1 bis 1 Gew.-%, bezogen auf das eingesetzte Ausgangs-Diamin.

Die Hydrierung wird durchgeführt bei einer Temperatur innerhalb des Bereichs von 150°C bis 210°C. Die genaue Wahl der Temperatur in einem bestimmten Falle hängt von der gewünschten Reaktionsgeschwindigkeit und dem angestrebten Gehalt an dem trans-trans-Isomeren ab. Im allgemeinen läuft die Reaktion um so schneller ab und ist der trans-trans-Gehalt des Endprodukts um so höher, je höher die Temperatur ist. Die Temperatur wird demnach im allgemeinen so gewählt, dass sie einen optimalen Kompromiss zwischen der Reaktionsdauer und dem Gehalt des trans-trans-Isomeren ergibt.

Der bei dem Verfahren gemäss der vorliegenden Erfindung angewandte Wasserstoff-Druck muss mindestens bei 172,4 bar (2500 psi) gehalten werden und liegt im allgemeinen zwischen 172,4 und 275,8 bar (2500 bis 4000 psi). Naturgemäss hängen die Drücke von den verwendeten Apparaturen ab. So können Drücke von 172,4 bis 551,6 bar (2500 bis 8000 psi) oder höher verwendet werden, wenn geeignete Hochdruck-Apparaturen zur Verfügung stehen. Im allgemeinen wurde gefunden, dass die Ausbeute mit steigendem Druck zunimmt.

Das Fortschreiten der Reaktion wird in einfacher Weise durch Beobachtung der von der Reaktionsmischung aufgenommenen Wasserstoff-Menge verfolgt, und die Hydrierung wird zu dem Zeitpunkt beendet, bei dem die theoretische Menge Wasserstoff absorbiert worden ist. Der Katalysator wird dann von der Lösung des reduzierten Materials abgetrennt, und letztere wird destilliert, um das Di-(4-aminocyclohexyl)methan daraus zu isolieren. Die Hydrierzeiten betragen im allgemeinen etwa 30 bis 90 min.

Falls erwünscht, kann auch Ammoniak verwendet werden, wie dies in einigen der oben genannten Patentschriften beschrieben wird (vgl. z.B. die US-PSen 3347917, 3636108 und 364452), obwohl dies zur Erzielung der Ergebnisse der vorliegenden Erfindung nicht erforderlich ist.

Im allgemeinen werden die Materialien miteinander vermischt und chargenweise dem Reaktionsgefäss zugeführt, jedoch könnte auch ein kontinuierliches Verfahren angewandt werden.

Die vorliegende Erfindung wird durch die folgenden Beispiele näher erläutert, ist jedoch nicht auf diese beschränkt. Hierin beziehen sich, sofern nicht anders vermerkt, sämtliche Angaben von Teilen und Prozentsätzen auf das Gewicht.

*Beispiele*

*Beispiele 1 bis 8:*

200 Teile n-Butylether, 200 Teile Di-(4-aminophenyl)methan und die in Tabelle 1 angegebene Menge des hydratisierten Rutheniumdioxids von Engelhard (Ruthenium-Gehalt 58,3%) wurden in einen Hochdruck-Autoklaven gefüllt. Der Autoklav wurde verschlossen und bei Raumtemperatur mit Wasserstoff bis zu einem Druck von etwa 275,8 bar (4000 psi) beschickt, und der Inhalt wurde auf die in Tabelle 1 angegebene Temperatur erhitzt. Die Reaktion wurde nach Beendigung des Wasserstoff-Verbrauchs noch 30 min auf der angegebenen Temperatur gehalten. Der Inhalt wurde dem Autoklaven bei Raumtemperatur entnommen und unter Vakuum filtriert. Die Produkte wurden auf ihren Gehalt an dem trans-trans-Isomeren analysiert und als flüssig oder nicht flüssig gekennzeichnet. Die erhaltenen Ergebnisse sind in Tabelle 1 aufgeführt.

*Tabelle 1*

| Beispiel | Katalysator Menge Ru-Metall | | Temp. | Gehalt trans-trans-I. | Flüssig | Ausbeute mittels HPLC |
|---|---|---|---|---|---|---|
| Nr. | Gew.-Tl. | Gew.-% | °C | % | | % |
| 1 | 3 | 0,81 | 150 | 18,5 | ja | 44 |
| 2 | 1 | 0,27 | 180 | 20,5 | ja | 95 |
| 3 | 1 | 0,27 | 196 | 22,3 | ja | 95 |
| 4 | 1 | 0,27 | 200 | 21,3 | ja | 64 |
| 5 | 1 | 0,27 | 202 | 26,4 | ja | 73 |
| 6 | 1 | 0,27 | 204 | 27,7 | ja | 74 |
| 7 | 1 | 0,27 | 208 | 30,7 | ja | 70 |
| 8*) | 1 | 0,27 | 215 | 49,1 | nein | 86 |

*) zu Vergleichszwecken; ausserhalb des Rahmens der vorliegenden Erfindung.

*Beispiele 9 bis 12:*

In ähnlicher Weise wie in den Beispielen 1 bis 8 wurden n-Butylether, Di-(4-aminophenyl)methan und hydratisiertes Rutheniumdioxid von Engelhard (Ruthenium-Gehalt 58,3%) in einen Hochdruck-Autoklaven gegeben. Der verwendete Katalysator wurde mit dem Ether vermischt und vor der Zugabe des Amins 1 h bei 200°C unter 137,9 bar (2000 psi) gehalten. Die Reaktionsbedingungen und Stoffmengen sind in Tabelle 2 aufgeführt, wobei die angegebenen Drücke während der gesamten Reaktion eingehalten wurden. Die Ausbeuten und Gehalte an dem trans-trans-Isomeren sind ebenfalls in Tabelle 2 aufgeführt.

*Tabelle 2*

| Beispiel | Temp. | Druck | | Katalysator Menge Ru-Metall | | Ether | Amin | $H_2$/Zeit | Ausbeute mittels HPLC | Gehalt trans-trans-I. |
|---|---|---|---|---|---|---|---|---|---|---|
| Nr. | °C | bar | (psi) | Gew.-Tl. | Gew.-% | Gew.-Tl. | Gew.-Tl. | min | % | % |
| 9 | 210 | 275,8 | (4000) | 0,37 | 0,11 | 200 | 200 | 70 | 90,3 | 36,3 |
| 10 | 210 | 275,8 | (4000) | 0,37 | 0,11 | 200 | 200 | 60 | 87,5 | 31,1 |
| 11 | 210 | 275,8 | (4000) | 0,56 | 0,11 | 150 | 300 | 30 | 86,6 | 26,3 |
| 12 | 210 | 275,8 | (4000) | 1,39 | 0,27 | 150 | 300 | 31 | 90,4 | 37,3 |
| 13 | 210 | 137,9 | (2000) | 0,56 | 0,11 | 150 | 300 | 90 | 40,0 | 30,7 |

**Patentansprüche**

1. Verfahren zur katalytischen Hydrierung von Di-(4-aminophenyl)methan zu flüssigem, 15 bis 40 Gew.-% des trans-trans-Isomeren enthaltenden Di-(4-aminocyclohexyl)methan, dadurch gekennzeichnet, dass Di-(4-aminophenyl)methan in Gegenwart eines trägerfreien Rutheniumdioxid-Katalysators unter einem Wasserstoff-Druck von mindestens 172,4 bar (2500 psi) und bei einer Temperatur von 150°C bis 210°C hydriert wird.

2. Verfahren nach Anspruch 1, dadurch gekennzeichnet, dass die Hydrierung in Gegenwart einer solchen Menge eines unter den Reaktionsbedingungen inerten Lösungsmittels durchgeführt wird, dass die Konzentration des Ausgangs-Diamins in der Reaktionsmischung von 5 bis 50 Gew.-% beträgt.

3. Verfahren nach Anspruch 2, dadurch gekennzeichnet, dass das Lösungsmittel n-Butylether ist.

4. Verfahren nach Anspruch 1, dadurch gekennzeichnet, dass der Katalysator in einer solchen Menge verwendet wird, dass die vorhandene Menge Ruthenium, berechnet als Rutheniummetall, mindestens 0,05 Gew.-%, bezogen auf die Menge des Ausgangs-Diamins, beträgt.

5. Verfahren nach Anspruch 4, dadurch gekennzeichnet, dass der Katalysator in einer solchen Menge verwendet wird, dass die vorhandene Menge Ruthenium, berechnet als Rutheniummetall, etwa 0,1 bis 3 Gew.-%, bezogen auf die Menge des Ausgangs-Diamins, beträgt.

6. Verfahren nach Anspruch 1, dadurch gekennzeichnet, dass der Wasserstoff-Druck von 172,4 bis 275,8 bar (2500 bis 4000 psi) beträgt.

7. Verfahren nach Anspruch 1, dadurch gekennzeichnet, dass der Wasserstoff-Druck von 172,4 bis 551,6 bar (2500 bis 8000 psi) beträgt.

8. Verfahren nach Anspruch 1, dadurch gekennzeichnet, dass die Reaktion in eine von 30 bis 90 min dauernde Zeitspanne durchgeführt wird.

**Revendications**

1. Procédé d'hydrogénation catalytique du di-(4-aminophényl)méthane en di-(4-aminocyclohexyl)méthane liquide contenant 15 à 40% en poids de l'isomère trans-trans, caractérisé en ce que le di-(4-aminophényl)méthane est hydrogéné en présence d'un catalyseur à base de dioxyde de ruthénium sans support sous une pression d'hydrogène d'au moins 172,4 bars (2500 lb/in²) et à une température de 150 à 210°C.

2. Procédé suivant la revendication 1, caractérisé en ce que l'hydrogénation est conduite en présence d'une quantité telle d'un solvant inerte dans les conditions réactionnelles que la concentration de la diamine de départ dans le mélange réactionnel s'élève à 5-50% en poids.

3. Procédé suivant la revendication 2, caractérisé en ce que le solvant est l'éther de n-butyle.

4. Procédé suivant la revendication 1, caractérisé en ce que le catalyseur est utilisé en une quantité telle que la quantité présente de ruthénium, exprimée en ruthénium métallique, soit d'au moins 0,05% en poids par rapport à la quantité de la diamine de départ.

5. Procédé suivant la revendication 4, caractérisé en ce que le catalyseur est utilisé en une quantité telle que la quantité présente de ruthénium, exprimée en ruthénium métallique, s'élève à environ 0,1-3% en poids par rapport à la quantité de la diamine de départ.

6. Procédé suivant la revendication 1, caractérisé en ce que la pression d'hydrogène s'élève à une valeur de 172,4 à 275,8 bars (2500 à 4000 lb/in²).

7. Procédé suivant la revendication 1, caractérisé en ce que la pression d'hydrogène s'élève à une valeur de 172,4 à 551,6 bars (2500 à 8000 lb/in²).

8. Procédé suivant la revendication 1, caractérisé en ce que la réaction est conduite pendant

un intervalle de temps d'une durée de 30 à 90 minutes.

## Claims

1. Process for the catalytic hydrogenation of di-(4-aminophenyl)methane to give liquid di-(4-aminocyclohexyl)methane containing 15 to 40% by weight of the trans-trans-isomer, characterized in that di-(4-aminophenyl)methane is hydrogenated in the presence of an unsupported ruthenium dioxide catalyst under a hydrogen pressure of at least 172.4 bar (2,500 psi) and at a temperature of from 150°C to 210°C.

2. Process according to Claim 1, characterized in that hydrogenation is carried out in the presence of such a quantity of a solvent which is inert under the reaction conditions that the concentration of the starting diamine in the reaction mixture is from 5 to 50% by weight.

3. Process according to Claim 2, characterized in that the solvant is n-butyl ether.

4. Process according to Claim 1, characterized in that the catalyst is used in such an amount that the amount of ruthenium present, calculated as ruthenium metal, is at least 0.05% by weight, based on the amount of starting diamine.

5. Process according to Claim 4, characterized in that the catalyst is used in such an amount that the amount of ruthenium present, calculated as ruthenium metal, is about 0.1 to 3% by weight, based on the amount of starting diamine.

6. Process according to Claim 1, characterized in that the hydrogen pressure is from 172.4 to 275.8 bar (2,500 to 4,000 psi).

7. Process according to Claim 1, characterized in that the hydrogen pressure is from 172.4 to 551.6 bar (2,500 to 8,000 psi).

8. Process according to Claim 1, characterized in that the reaction is carried out for a period of from 30 to 90 min.